## Europäisches Patentamt

## European Patent Office

## Office européen des brevets

(11) Veröffentlichungsnummer: **0 020 288**
**A1**

(12) **EUROPÄISCHE PATENTANMELDUNG**

(21) Anmeldenummer: **80730036.3**

(22) Anmeldetag: **22.05.80**

(51) Int. Cl.³: **A 61 B 5/04,** G 01 N 27/30,
B 43 K 8/00

(30) Priorität: **25.05.79 DE 2921858**

(43) Veröffentlichungstag der Anmeldung: **10.12.80**
**Patentblatt 80/25**

(84) Benannte Vertragsstaaten: **AT CH DE FR GB IT LI NL SE**

(71) Anmelder: **BIOTRONIK Mess- und Therapiegeräte GmbH & Co Ingenieurbüro Berlin, Sleversufer 8, D-1000 Berlin 47 (DE)**

(72) Erfinder: **Nagel, Joachim, Dr., Dipl.-Phys., Schobertweg 3A, D-8520 Erlangen (DE)**

(74) Vertreter: **Christiansen, Henning, Dipl.-Ing., Unter den Eichen 108a, D-1000 Berlin 45 (DE)**

(54) **Elektrolytelektrode zur Ableitung bioelektrischer Signale.**

(57) Bei dieser Elektrode zur Ableitung bioelektrischer Signale über die Hautoberfläche mit einem Reservoir für einen flüssigen Elektrolyten und einer für diesen durchlässigen Spitze ist ein im wesentlichen abgeschlossenes, das Reservoir bildendes Gehäuse (1) mit einem saugfähigen Material (2) ausgefüllt.

Dipl.-Ing. Henning Christiansen
Patentanwalt

zugelassener Vertreter beim Europäischen Patentamt

Unter den Eichen 108a
D 1000 Berlin 45
Telefon 030-831 40 80

**0020288**

BIOTRONIK Meß- und Therapiegeräte
GmbH & Co Ingenieurbüro Berlin
1000 Berlin

21. Mai 1979

B79.9-EU

---

Elektrolytelektrode zur Ableitung bioelektrischer Signale

---

B e s c h r e i b u n g

Die Erfindung betrifft eine Elektrolytelektrode zur Ableitung bioelektrischer Signale über die Hautoberfläche mit einem Reservoir für einen flüssigen Elektrolyten und einer für diesen durchlässigen Spitze.

/2

Eine derartige Elektrode dient beispielsweise dazu, durch frei bewegliches Kontaktieren der Hautoberflache des Patienten ein kurzfristiges, einen ersten Eindruck vermittelndes EKG abzuleiten oder günstige Ableitungspositionen für festanzubringende Elektroden aufzusuchen und ersetzt dabei die für kurzzeitige Ableitungen benutzten Saugelektroden.

Bei einer bekannten Elektrode der vorgenannten Gattung ("Manual e.c.g. electrode" in: Med. & Biol. Eng. & Comput., 1979, 17, 141) ist ein röhrenförmiger offener Hohlbehälter als Reservoir für die Salzlösung vorgesehen, dessen untere Öffnung entweder durch eine Baumwoll- oder eine Faserspitze abgeschlossen ist. Die Flüssigkeit, in die ein freies Drahtende der Elektrodenzuleitung eintaucht, ist im übrigen innerhalb des Behälters frei beweglich.

Nachteilig ist dabei, daß diese Elektrode nicht lage- und beschleunigungsunabhangig arbeitet, da stets gleichzeitig sowohl das Frontende der Elektrode als auch der Elektrodenanschlußdraht von Flüssigkeit benetzt sein müssen. Dazu kommt, daß der Flüssigkeitsaustritt aus der Spitze relativ unkontrolliert erfolgt, da dieser wegen des ungehinderten Zutritts der Flüssigkeit im wesentlichen durch den Zustand der Spitze bestimmt wird, der jedoch bei der Benutzung Veränderungen unterworfen ist. Wird die Elektrode frei in der Luft gehalten, so besteht die Gefahr, daß Flüssigkeit heraustropft. Wegen des großen Flüssigkeitsverbrauchs ist ein häufiges Nachfüllen erforderlich. Die bekannte Elektrode ist auch aufgrund ihrer Bauform umständlich handhabbar.

/3

Der in Anspruch 1 angegebenen Erfindung liegt die Aufgabe zugrunde, die vorgenannten Nachteile zu beseitigen und eine Elektrode anzugeben, die einfacher handhabbar und für einen längeren Zeitraum wartungsfrei ist.

Besonders vorteilhaft ist bei der vorgenannten Elektrode, daß sie preiswert - vorzugsweise unter Verwendung von für Schreibstifte in großen Serien hergestellten Bauelementen - erzeugbar und jederzeit betriebsbereit ist. Durch die Füllung aus Fasermaterial, das auch bei teilweise entleerter Elektrode noch feucht bleibt, ist eine sichere Kontaktierung vom der Spitze entgegengesetzen Ende her gewahrleistet, so daß keine Drahtdurchführungen innerhalb des Reservoirs erforderlich sind. Die Kontaktierungsmittel für das Fasermaterial bilden vorzugsweise ihrerseits gleichzeitig das Gegenstück für den Anschlußclip einer Elektrodenzuleitung, so daß auch in dieser Beziehung eine einfache Handhabung möglich ist.

Durch die Kapillarwirkung der Faserspitze im Zusammenwirken mit dem saugfähigen Material des Reservoirs ist ein gleichmäßiger Fluß des Elektrolyten bei der Benutzung gewährleistet.

Die erfindungsgemäße Elektrode ist günstig anwendbar für die Ableitung des fetalen Elektrokardiogramms, wo oft eine große Zahl von Elektrodenpositionen überprüft werden muß, bevor Klebeelektroden permanent fixiert werden können.

Vorteilhafte Weiterbildungen der Erfindung sind in den Unteransprüchen angegeben. Ein Ausführungsbeispiel der Erfindung einschließlich zweier Varianten ist in den Figuren 1 bis 3 dargestellt und wird nachfolgend näher beschrieben. Es zeigen:

Fig. 1    eine Gesamtdarstellung des Ausführungsbeispiels der Erfindung in vergrößerter Darstellung,

Fig. 2    eine erste Variante der Ausführung gemäß Fig. 1 und

Fig. 3    eine zweite Variante der Ausführung gemäß Fig. 1.

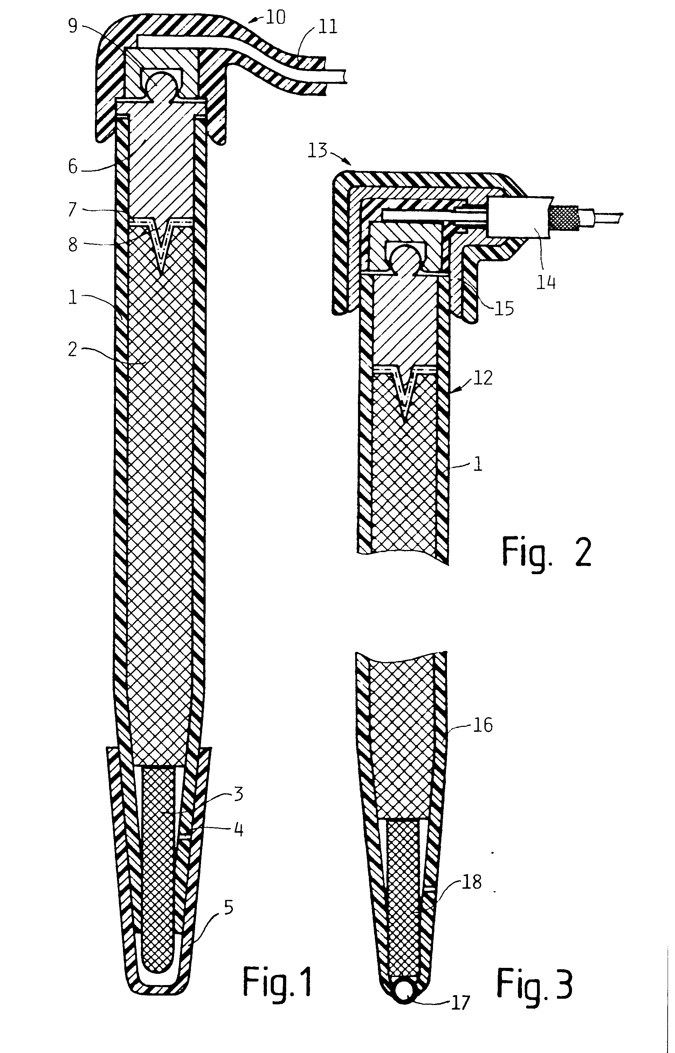

Bei der in Fig. 1 dargestellten Ausführungsform hat die handgeführte Elektrode die Form eines Schreibstiftes, wobei die äußere Form durch eine Hülse 1 aus nicht leitendem Stoff bestimmt wird. Diese Hülse bildet das Reservoir für einen flüssigen Elektrolyten (vorzugsweise eine gesättigte NaCl-Lösung), mit dem eine Füllung 2 aus saugfähigem Fasermaterial getränkt ist.

Der Elektrolyt enthält für Markierungszwecke einen abwaschbaren Farbstoffzusatz. Dadurch läßt sich eine gefundene günstige Ableitungsposition fixieren, so daß sie zum nachfolgenden Anbringen einer herkömmlichen Klebeelektrode ohne Schwierigkeiten auffindbar ist.

An die Füllung 2 schließt sich im Bereich des sich verjüngenden Endes der Hülse 1 eine Tastspitze 3 aus Fasermaterial an, die mit der saugfähigen Füllung in Kontakt steht. Um einen Druckausgleich innerhalb der im übrigen bis auf einen Durchlaß für die Tastspitze 3 hermetisch geschlossene Hülse 1 mit zunehmenden Verbrauch des Elektrolyten zu ermöglichen, ist eine Belüftungsbohrung 4 vorgesehen, die, wenn das schreibende Ende der Elektrode durch eine Kappe 5 verschlossen ist, ebenfalls abgedeckt ist, so daß ein Austrocknen der Elektrode verhindert ist. Die Schreibspitze 3 aus Fasermaterial kann in ihrer Ausführung derjenigen üblicher Filzschreiber entsprechen, wobei ein gleichmäßiger Fluß des Elektrolyten und damit eine gleichmäßige Benetzung der Hautoberfläche des Patienten zur Sicherstellung guten elektrischen Kontaktes gewährleistet ist.

Das hintere Ende der Hülse 1 ist durch einen Elektrodenkörper 6 verschlossen, der aus Kunstoff bestehen kann, dessen Oberfläche mit Metall beschichtet ist, wobei die Beschichtung durch Galvanisieren aufgebracht wird. Als Beschichtungsmaterial wird vorzugsweise Silber verwendet, so daß sich bei Benutzung von NaCl als Elektrolyt eine wirksame Elektrodenoberfläche 7 aus Silberchlorid bildet. Eine an dem Elektrodenkörper 6 vorgesehene, in die Füllung hineinragende Spitze 8 vergrößert den Kontaktierungsbereich zwischen Elektrodenoberfläche 7 und Füllung 2. Die Spitze 8 übt dabei Druck auf das Füllmaterial aus, wobei die aufgrund der Elastizität des Füllmaterials ausgeübte Kraft ebenfalls zur Verbesserung des elektrischen Kontakts dient.

Am äußeren Ende des Elektrodenkörpers ist ein Druckknopfanschluß 9 vorgesehen, der in seiner Ausführung den Anschlüssen ortsfester Elektroden entspricht, so daß der aufgesetzte Anschlußstecker 10 für die Elektrodenzuleitung 11 ohne Mühe spater an eine übliche Klebeelektrode angeschlossen werden kann, sobald ein geeigneter Ableitungsort gefunden ist.

Bei der in Fig. 2 dargestellten Variante der Ausführungsform der erfindungsgemäßen Elektrode ist die Außenoberfläche der Hülse 1 mit einem galvanischen, aufgedampften oder als leitenden Farbanstrich aufgetragenen Oberzug 12 aus leitendem Material versehen, der als Abschirmung dient. Ein derartiger Oberzug läßt sich ohne Schwierigkeiten an den Oberflächen der Hülsen herkömmlicher Schreibgeräte anbringen und kann durch einen entsprechend ausgebildeten abgeschirmten Stecker 13 für eine abgeschirmte Zuleitung 14 durch eine angepaßte Abschirmhülse 15 direkt kontaktiert werden, so daß eine kontinuierliche Abschirmung ohne Beeinträchtigung des abgeleiteten Signals durch eingestreute Störsignale gewährleistet ist. Der Oberzug 12 weist seinerseits einen weiteren Oberzug auf, der aus Isoliermaterial besteht, um Störungen durch Berührung zu vermeiden. Dieser zweite Oberzug ist in der Zeichnung nicht dargestellt, kann aber in üblicher Weise durch Tauchen, Lackieren oder ein aufgeschrumpftes Material gebildet werden, wobei der Kontaktierungsbereich der Abschirmung für den Stecker 13 freigelassen ist.

In Fig. 3 ist eine Variante der Tastspitze der erfindungsgemäßen Elektrode wiedergegeben, wobei das kegelförmige Ende 16 der Hülse 1 als Lager für eine Kugel 17 dient, die aus Kunstoff oder Metall gefertigt sein kann. Die Verbindung zwischen der - in Fig. 3 nicht dargestellten - Füllung wird durch einen Einsatz 18 aus Fasermaterial gebildet, der mit seinen Enden einerseits an der Kugel 17 und andererseits an der Füllung anliegt. Bei dieser Ausführungsvariante wird die Körperoberfläche durch die Kugel benetzt und die elektrische Verbindung über den an der Kugeloberfläche befindlichen leitenden Film bzw. das Leitermaterial der Kugel herstellt.

*  *  *  *  *

Patentansprüche

1. Elektrolytelektrode zur Ableitung bioelektrischer Signale über die Hautoberfläche mit einem Reservoir für einen flüssigen Elektrolyten und einer für diesen durchlässigen Spitze, d a d u r c h   g e k e n n z e i c h n e t , daß ein im wesentlichen abgeschlossenes, das Reservoir bildendes Gehause (1) mit einem saugfähigen Material (2) ausgefüllt ist.

2. Elektrolytelektrode nach Anspruch 1, d a d u r c h   g e k e n n z e i c h n e t , daß es sich bei dem saugfähigen Material (2) um ein Fasermaterial handelt.

3. Elektrolytelektrode nach einem der Ansprüche 1 oder 2, d a d u r c h   g e k e n n z e i c h n e t , daß ein zum Anschließen der Elektrodenzuleitung (11, 14) dienender Elektrodenkörper (6) mit leitfähiger Oberfläche eine in das saugfahige Material (2) hineinragende Spitze (8) aufweist.

4. Elektrolytelektrode nach Anspruch 3, d a d u r c h   g e k e n n z e i c h n e t , daß der Elektrodenkörper (6) eine aus Silber bestehende Oberfläche aufweist.

5. Elektrolytelektrode nach einem der vorangehenden Ansprüche, d a d u r c h   g e k e n n z e i c h n e t ,

daß zur Benetzung der Hautoberfläche mit der elektrolytischen Flüssigkeit eine Kugel (17) oder Faserspitze (3)
vorgesehen ist.

6.      Elektrolytelektrode nach einem der vorangehenden
Ansprüche, d a d u r c h   g e k e n n z e i c h n e t ,
daß das Gehäuse mit der Spitze (3) die Form eines Schreibgerätes aufweist und die Spitze (3) mit einer an das Gehäuse angepaßten Kappe (5) verschließbar ist.

7.      Elektrolytelektrode nach einem der vorangehenden Ansprüche, d a d u r c h   g e k e n n z e i c h n e t , daß
eine Abschirmung durch einen das Gehäuse (1) umgebenden
metallischen Überzug (12) gebildet ist, der seinerseits
wieder einen isolierenden Überzug aufweist.

8.      Elektrolytelektrode nach Anspruch 7, d a d u r c h
g e k e n n z e i c h n e t ,   daß bei einem aus Kunststoff bestehenden Gehäuse (1) der Überzug (12) galvanisch
oder als leitfähige Farbe aufgebracht bzw. aufgedampft
ist.

9.      Elektrolytelektrode nach einem der vorangehenden Ansprüche, d a d u r c h   g e k e n n z e i c h n e t , daß
der Anschlußkontakt (9) an den Elektrodenstecker (11, 13)
für eine spater fest anzuschließenden Elektrode angepaßt
ist.

10. Elektrolytelektrode nach einem der vorangehenden Ansprüche, d a d u r c h   g e k e n n z e i c h n e t , daß
dem   flüssigen Elektrolyten ein abwaschbarer Farbstoff
zugesetzt ist.

\* \* \* \* \*

Fig.1

Fig. 2

Fig. 3

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | MEDICAL AND BIOLOGICAL ENGINEERING AND COMPUTING, vol. 17, Nr. 1, Januar 1979 STEVENAGE (GB) M.G. PEPPER et al.: "Manual e.c.g. electrode", Seite 141 <br><br> * Seite 141 * <br><br> --- | 1,2, 4,5 |
| | US - A - 3 989 036 (S. SASAMORI/ DIA MEDICAL SYSTEM CO. LTD.) <br><br> * Zusammenfassung; Spalte 3, Zeilen 6-39; Spalte 4, Zeilen 13-27, 36-45 und 56-63; Abbildungen 1,3,5,7 * <br><br> --- | 1,2, 4,9 |
| | US - A - 4 033 334 (J.C. FLETCHER/ NASA) <br><br> * Zusammenfassung; Spalte 2, Zeile 12 bis Spalte 3, Zeile 29 und die Abbildungen * <br><br> --- | 1,9 |
| | US - A - 3 826 245 (H. FUNFSTUCK/ STATHAM INSTRUMENTS INC.) <br><br> Zusammenfassung; Spalte 2, Zeile 33 bis Spalte 3, Zeile 13 und Abbildungen 2 und 3 * <br><br> --- | 1,3,9 |
| | US - A - 3 540 434 (A.H. FREY/USA SECRETARY OF THE NAVY) <br><br> * Zusammenfassung; Spalte 1, Zeilen 35-41 und Abbildung 1 * <br><br> --- | 7,8 |
| | US - A - 1 550 048 (S. RUBEN) <br><br> * Seite 1, Zeilen 31-60; Seite 1, Zeile 101 bis Seite 2, Zeile 26 und Abbildungen 1 und 2 * <br><br> --- | 5,6 |

KLASSIFIKATION DER ANMELDUNG (Int.Cl.)

A 61 B 5/04
G 01 N 27/30
B 43 K 8/00

RECHERCHIERTE SACHGEBIETE (Int Cl.)

A 61 B 5/04
5/10
G 01 N 27/30
B 43 K 8/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

./..

Der vorliegende Recherchenbericht wurde für alle Patentansprüche erstellt.

| Recherchenort | Abschlußdatum der Recherche | Prüfer |
|---|---|---|
| Den Haag | 2. September 1980 | RIEB |

EPA form 1503.1 06.78

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | <u>FR - A - 2 206 194</u> (G. WAGNER/ PELIKAN-WERKE GmbH) <br><br> * Seite 1, Zeile 37 bis Seite 2, Zeile 1; Seite 2, Zeile 34 bis Seite 3, Zeile 21 und Abbildung 2 * <br><br> --- | 5,6 |
| | <u>US - A - 3 810 459</u> (G.O. BECKER/ AMERICAN OPTICAL CORP.) <br><br> * Zusammenfassung; Spalte 1, Zeile 45 bis Spalte 2, Zeile 6; Spalte 3, Zeile 42 bis Spalte 4, Zeile 29 und Abbildungen 1-3 * | 10 |

**KLASSIFIKATION DER ANMELDUNG (Int.Cl. 3)**

**RECHERCHIERTE SACHGEBIETE (Int. Cl. 3)**